# EUROPEAN PATENT APPLICATION

(11) **EP 3 570 022 A1**
(43) Date of publication of application: **20.11.2019**
(21) Application number: 18185933.1
(22) Date of filing: 27.07.2018
(51) Int. Cl.: G01N 33/00, G01N 1/22, G08B 21/16

(54) **GAS DETECTION APPARATUS**

(30) Priority: 16.05.2018 KR 20180055760
(71) Applicant: GRIB CO., LTD., Seocho-gu Seoul (KR)
(72) Inventor: JUNG, Yeon Kyu, Seoul (KR); YOO, Jae Ho, Seocho-gu, Seoul (KR); CHOI, Seung Wook, Seoul (KR)
(74) Representative: Glawe, Delfs, Moll

(57) **Abstract**

Provided is a gas detection apparatus including: a main body (2) in which a gas sensor (20) is mounted; an inlet part (3) disposed under the main body (2) to introduce a gas present in a detection space; and a plurality of inlet holes (31) formed to pass through the inlet part, wherein, in each of the inlet holes (31), an area of an inlet (311) facing the detection space is greater than that of an outlet (312) facing the main body (2).

## Description

This application claims priority from Korean Patent Application No. 10-2018-0055760 filed on May 16, 2018 in the Korean Intellectual Property Office, the disclosure of which is incorporated herein by reference in its entirety.

### BACKGROUND

### 1. Field of the Disclosure

An inventive concept relates to a gas detection apparatus into which gases present in a predetermined detection space are introduced.

### 2. Description of the Related Art

A gas detection apparatus detects an emergency situation, such as an outbreak of fire, generation of harmful gases, and the like, using gases present in a predetermined detection space. For example, the gas detection apparatus may detect a fire outbreak by using gases diffusing in the detection space at a beginning of the outbreak of fire.

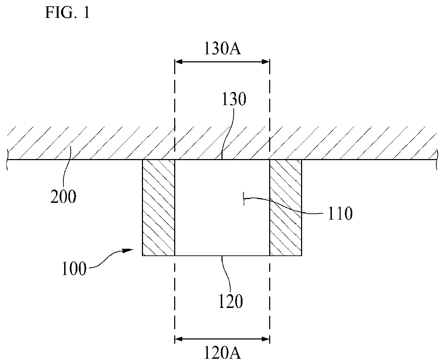

FIG. 1 is a schematic cross-sectional side view illustrating a gas detection apparatus according to a conventional technology.

Referring to FIG. 1, a gas detection apparatus 100 according to the conventional technology is installed on a ceiling 200 of a detection space. The gas detection apparatus 100 according to the conventional technology includes an inlet path 110 through which gases are introduced. The gas detection apparatus 100 according to the conventional technology transfers the gases introduced through the inlet path 110 to a gas sensor (not shown). The gas sensor analyzes components of the gases.

The inlet path 110 includes an inlet 120 facing the detection space and an outlet 130 facing the gas sensor. The gases introduced through the inlet 120 are transferred to the gas sensor through the outlet 130.

Here, in the gas detection apparatus 100 according to the conventional technology, an area 120A of the inlet 120 is the same size as an area 130A of the outlet 130. That is, the inlet path 110 is formed to have a constant area in a flow direction of the gases. Accordingly, in the gas detection apparatus 100 according to the conventional technology, since an amount of the introduced gases present in the detection space is small, capability for detecting an emergency situation using the introduced gases is low.

### SUMMARY

Aspects of the inventive concept provide a gas detection apparatus capable of increasing an amount of gases which are present in a detection space and are to be introduced into the gas detection apparatus.

It should be noted that objects of the present invention are not limited to the above-described objects, and other objects of the present invention will be apparent to those skilled in the art from the following descriptions.

According to aspects of the inventive concept, an inventive concept may have the following configuration.

A gas detection apparatus according to the inventive concept may include: a main body in which a gas sensor is mounted; an inlet part disposed under the main body to introduce a gas present in a detection space; and a plurality of inlet holes formed to pass through the inlet part. In each of the inlet holes, an area of an inlet facing the detection space may be greater than that of an outlet facing the main body.

### BRIEF DESCRIPTION OF THE DRAWINGS

The above and other aspects and features of the present disclosure will become more apparent by describing in detail exemplary embodiments thereof with reference to the attached drawings, in which:
FIG. 1 is a schematic cross-sectional side view illustrating a gas detection apparatus according to a conventional technology;
FIG. 2 is schematic perspective view illustrating a gas detection apparatus according to an inventive concept;
FIG. 3 is a conceptual side view illustrating the gas detection apparatus according to the inventive concept which is installed on a ceiling;
FIG. 4 is a schematic cross-sectional side view illustrating a part of an inlet part of the gas detection apparatus according to the inventive concept and taken along line I-I of FIG. 3;
FIG. 5 is a schematic bottom view illustrating the inlet part of the gas detection apparatus according to the inventive concept;
FIG. 6 is an enlarged view illustrating a portion II of FIG. 5;
FIG. 7 is a schematic cross-sectional side view illustrating a part of an inlet part of a gas detection apparatus according to a modified embodiment of the inventive concept and taken along line I-I of FIG. 3;
FIG. 8 is a schematic perspective bottom view illustrating the gas detection apparatus according to the modified embodiment of the inventive concept;
FIG. 9 is a schematic bottom view illustrating the inlet part of the gas detection apparatus according to the modified embodiment of the inventive concept;
FIG. 10 is a conceptual side view illustrating the gas detection apparatus according to the modified embodiment of the inventive concept which is installed on a ceiling;
FIG. 11 is a schematic block diagram illustrating an image capture part and peripheral parts of a gas detection apparatus according to another inventive concept;
FIG. 12 is a bottom view illustrating an inlet part in which the image capture part and the peripheral parts are installed in the gas detection apparatus according to another inventive concept;
FIG. 13 is a schematic cross-sectional plan view illustrating a main body of the gas detection apparatus according to another inventive concept and taken along line III-III of FIG. 10;
FIG. 14 is a schematic plan view illustrating a protection part which is coupled to an upper end of the inlet part in the gas detection apparatus according to another inventive concept;
FIG. 15 is a schematic plan view illustrating the protection part and a wire accommodated in the protection part in the gas detection apparatus according to another inventive concept;
FIG. 16 is a block diagram illustrating components of a gas detection apparatus according to still another inventive concept;
FIG. 17 is a graph showing a change in amount of gas over time detected by a gas sensor of the gas detection apparatus according to still another inventive concept when a fire breaks out;
FIG. 18 is a schematic block diagram illustrating a gas detection apparatus, a relay part, and an external server according to still another inventive concept;
FIG. 19 is a view illustrating an example in which information is transmitted and received between the gas detection apparatus, the relay part, and the external server according to still another inventive concept when a fire breaks out in a detection space; and
FIG. 20 is a flowchart for describing a process flow for detecting an outbreak of fire using the gas detection apparatus according to still another inventive concept.

### DETAILED DESCRIPTION OF THE EMBODIMENTS

Hereinafter, embodiments of a gas detection apparatus according to the inventive concept will be described in detail with reference to the accompanying drawings.

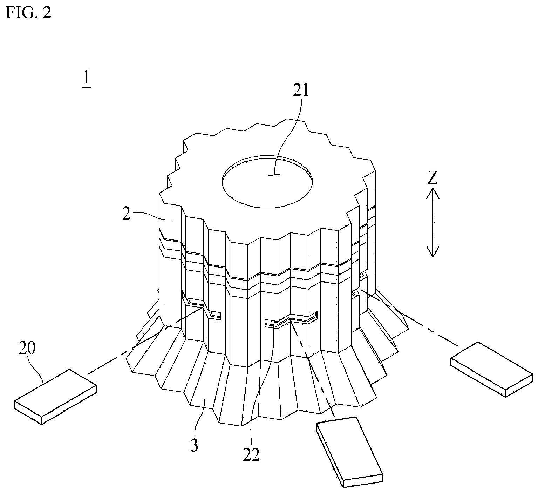

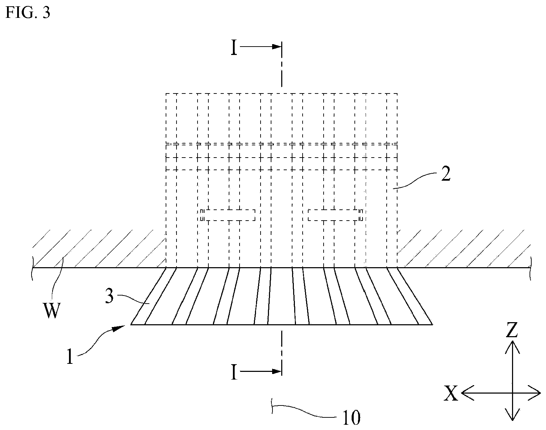

Referring to FIGS. 2 to 4, a gas detection apparatus 1 according to the inventive concept detects an emergency situation, such as an outbreak of fire, generation of a harmful gas, and the like, using a gas present in a detection space 10 (illustrated in FIG. 3). The detection space 10 may be in one of various places such as a house, a company, and a factory.

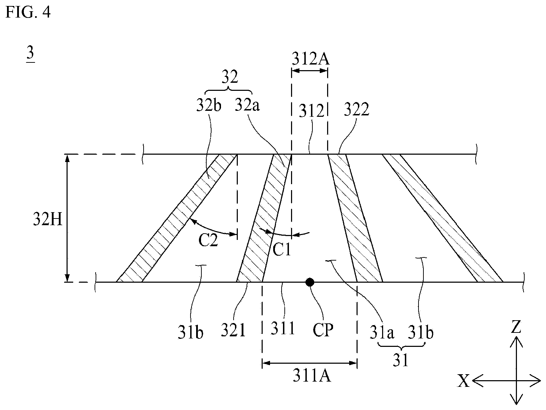

The gas detection apparatus 1 according to the inventive concept includes a main body 2 in which gas sensors 20 are mounted, and an inlet part 3 into which a gas present in the detection space 10 is introduced. The inlet part 3 is disposed under the main body 2 in a vertical direction (a Z-axis direction). A plurality of inlet holes 31 (illustrated in FIG. 4) are formed in the inlet part 3. The inlet holes 31 are formed to pass through the inlet part 3. Accordingly, a gas present in the detection space 10 may flow into the inlet part 3 through the inlet holes 31, and may be transferred to the gas sensor 20 mounted in the main body 2 through the inlet part 3. In the inlet holes 31, an area of each of inlets 311 (illustrated in FIG. 4) facing the detection space 10 is greater than that of each of outlets 312 (illustrated in FIG. 4) facing the main body 2. The area of each of the inlets 311 and the outlets 312 is an area of a cross section thereof in a horizontal direction (an X-axis direction) perpendicular to the vertical direction (the Z-axis direction). That is, the area 311A (illustrated in FIG. 4) of each of the inlets 311 is greater than the area 312A of each of the outlets 312 (illustrated in FIG. 4) in the horizontal direction (the X-axis direction).

Accordingly, the gas detection apparatus 1 according to the inventive concept may have the following functional effects.

First, in the gas detection apparatus 1 according to the inventive concept, since the area 311A of each of the inlets 311 is greater than the area 312A of the outlet 312, an area of an entrance into which a gas from the detection space 10 is introduced can increase. Accordingly, in the gas detection apparatus 1 according to the inventive concept, since a gas can be introduced from a wider region, an amount of gas introduced through the inlets 311 can increase.

Second, in the gas detection apparatus 1 according to the inventive concept, the area 312B of each of the outlets 312 is less than the area 311A of each of the inlets 311, the area decreases in a process in which a gas flows upward along each of the inlet holes 31. Accordingly, in the gas detection apparatus 1 according to the inventive concept, since a flow speed of a gas increases in the process in which the gas flows upward along the inlet holes 31 according to the Bernoulli's theorem, an amount of gas transferred to the gas sensor 20 installed in the main body 2 through the inlet part 3 can increase. Accordingly, the gas detection apparatus 1 according to the inventive concept can be realized such that the gas sensor 20 can more quickly and accurately identify whether an emergency situation occurs in the detection space 10 by analyzing a gas introduced through the inlet part 3. In addition, in the gas detection apparatus 1 according to the inventive concept, an increase in flow speed of a gas flowing upward along the inlet holes 31 can induce a suction force by which the gas is introduced into the inlets 311. Accordingly, the gas detection apparatus 1 according to the inventive concept can increase an amount of gas introduced through the inlets 311.

Hereinafter, the main body 2 and the inlet part 3 will be described in detail with reference to the accompanying drawings.

Referring to FIGS. 2 to 4, the main body 2 supports the gas sensors 20. Since the gas sensors 20 are mounted in the main body 2, the gas sensors 20 may be supported by the main body 2. A plurality of gas sensors 20 may be mounted in the main body 2. In this case, the gas sensors 20 may be mounted in the main body 2 to be spaced apart from each other along an outer surface of the main body 2. The main body 2 may be formed in an entirely hexagonal prism shape, but is not limited thereto, and may also be formed in another shape such as a cylindrical shape or a polygonal prism shape as long as the gas sensor 20 is mountable in the main body 2.

The main body 2 may include a transfer hole 21 (illustrated in FIG. 2).

The transfer hole 21 is formed to be connected to the inlet holes 31. Accordingly, a gas introduced into the inlet part 3 through the inlet holes 31 may be introduced into the main body 2 through the transfer hole 21. The gas sensor 20 may identify whether an emergency situation occurs in the detection space 10 using the gas flowing along the transfer hole 21. The transfer hole 21 may be formed to be connected to the outlets 312 of the inlet holes 31.

The transfer hole 21 may be formed to pass through the main body 2. The transfer hole 21 may be formed to pass through the main body 2 in the vertical direction (the Z-axis direction). In this case, a lower end of the transfer hole 21 may be connected to the outlets 312 of the inlet holes 31 and an upper end thereof may be connected to a gas processing system (not shown). The gas processing system may discharge a gas discharged from the transfer hole 21 to the outside. The gas processing system may also discharge the gas discharged from the transfer hole 21 after performing a predetermined treatment process such as a harmful material reduction process for the gas.

The transfer hole 21 may be formed in an entirely cylindrical shape, but is not limited thereto, and may also be formed in another shape such as a polygonal prism shape as long as the transfer hole 21 is connectable to the inlet holes 31 so that a gas may be introduced into the transfer hole 21.

The main body 2 may include an installation hole 22 (illustrated in FIG. 2).

The installation hole 22 is formed to be connected to the transfer hole 21. The gas sensor 20 may be inserted into the installation hole 22. Since the gas sensor 20 is inserted into the installation hole 22, the gas sensor 20 may be mounted in the main body 2. The gas sensor 20 may be inserted into the installation hole 22 such that a part of the gas sensor 20 is positioned in the transfer hole 21. Accordingly, since the gas sensor 20 directly comes into contact with a gas flowing along the transfer hole 21, whether an emergency situation occurs in the detection space 10 may be determined by analyzing the gas flowing along the transfer hole 21. The remaining part of the gas sensor 20 may be inserted into the installation hole 22.

The installation hole 22 may be formed to pass through the main body 2. The installation hole 22 may be formed to pass through the main body 2 in the horizontal direction (the X-axis direction) so as to be connected to the transfer hole 21. The installation hole 22 may be formed in an entirely tetragonal plate shape, but is not limited thereto, and may also be formed in anther shape as long as the gas sensor 20 is insertable into the installation hole 22 and mountable in the main body 2. In this case, the installation hole 22 may be formed in a shape corresponding to an outer surface of the gas sensor 20.

A plurality of installation holes 22 may also be formed in the main body 2. In this case, the installation holes 22 may be formed to pass through the main body 2 at positions spaced apart from each other along the outer surface of the main body 2. The gas sensors 20 may be inserted into the installation holes 22. In this case, the gas sensors 20 may analyze different components of gases.

Referring to FIGS. 2 to 4, the inlet part 3 is for introducing a gas which is present in the detection space 10. The inlet part 3 is installed under the main body 2 in the vertical direction (the Z-axis direction). Accordingly, the gas may flow into the inlet part 3 and flow to the gas sensor 20 mounted in the main body 2 through the inlet part 3. The inlet part 3 may be coupled to the main body 2 to be disposed under the main body 2. The inlet part 3 and the main body 2 may also be integrally formed.

The inlet part 3 includes the inlet holes 31.

The inlet holes 31 are formed to pass through the inlet part 3. The inlet holes 31 may be formed to pass through the inlet part 3 at positions spaced apart from each other. The inlet holes 31 may be connected to the transfer hole 21 formed in the main body 2. Accordingly, a gas introduced into the inlet holes 31 may flow into the transfer hole 21. The inlet part 3 may be disposed such that lower sides of the inlet holes 31 face the detection space 10. The inlet part 3 may protrude downward from a ceil W (illustrated in FIG. 3) of the detection space 10. In this case, the main body 2 may be coupled to the ceil W to be positioned inside the ceil W.

The inlet holes 31 include the inlets 311 and the outlets 312.

The inlet 311 faces the detection space 10. The inlet 311 corresponds to an entrance through which a gas present in the detection space 10 is introduced into the inlet part 3. The inlet 311 may correspond to a lower end of the inlet hole 31 in the vertical direction (the Z-axis direction).

The outlet 312 faces the main body 2. The outlet 312 corresponds to an exit through which a gas introduced through the inlet hole 31 flows into the main body 2. The outlet 312 may correspond to an upper end of the inlet hole 31 in the vertical direction (the Z-axis direction). The outlet 312 may be connected to the transfer hole 21.

An area of the outlet 312 is less than that of the inlet 311. In this case, the area 311A of the inlet 311 is greater than the area 312A of the outlet 312. Accordingly, in the gas detection apparatus 1 according to the inventive concept, since the area of the entrance for introducing a gas from the detection space 10 increases, a larger amount of gas is introduced from a wider region of the detection space 10. In addition, in the gas detection apparatus 1 according to the inventive concept, since a flow speed of a gas increases in a process in which the gas flows upward along the inlet holes 31 according to the Bernoulli's theorem, an amount of gas transferred to the gas sensor 20 installed in the main body 2 through the inlet part 3 increases, and thus it can be more accurately and rapidly identified whether an emergency situation occurs in the detection space 10.

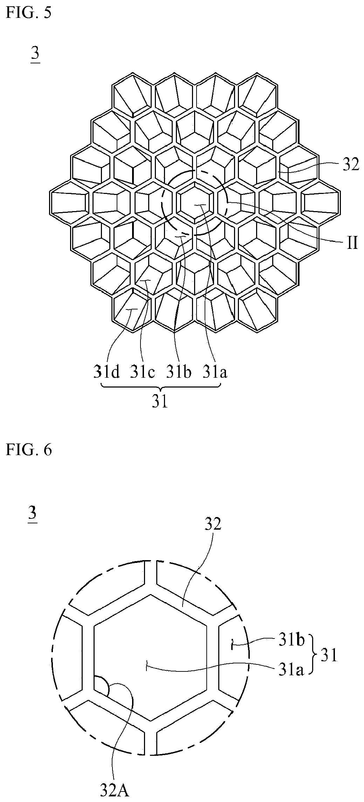

Since the inlet part 3 is formed such that the area thereof increases as the inlet part 3 extends downward, the inlet holes 31 formed further outward may be obliquely formed further outward as illustrated in FIGS. 3 and 5. The term "outward" refers to a direction toward the outside in a radial direction from the inlet hole 31a (hereinafter, central inlet hole 31a) disposed in a central portion among the inlet holes 31. The plurality of inlet holes 31 may be disposed along hexagonal shapes disposed around a center point CP (illustrated in FIG. 4) of the central inlet hole 31a and have different areas. The center point CP of the central inlet hole 31a is based on a lower end positioned at a lowermost side in the vertical direction (the Z-axis direction). For example, with respect to the center point CP of the central inlet hole 31a, a plurality of first inlet holes 31b may be disposed to surround the central inlet hole 31a along a first hexagonal shape, a plurality of second inlet holes 31c may be disposed to surround the first inlet holes 31b along a second hexagonal shape having an area greater than that of the first hexagonal shape, and a plurality of third inlet holes 31d may be disposed to surround the second inlet holes 31c along a third hexagonal shape having an area greater than that of the second hexagonal shape. In this case, the third inlet holes 31d may be obliquely formed further outward as compared to the second inlet holes 31c. The second inlet holes 31c may be obliquely formed further outward as compared to the first inlet holes 31b. The first inlet holes 31b may be obliquely formed further outward as compared to the central inlet hole 31a. The central inlet hole 31a may be formed downward in a vertical direction to be parallel to the vertical direction (the Z-axis direction).

Accordingly, in the gas detection apparatus 1 according to the inventive concept, since the inlet holes 31 disposed further outward are obliquely formed further outward, a gas may be introduced from a wider region in the detection space 10, and thus an amount of gas introduced through the inlets 311 can further increase.

In FIG. 5, the third inlet holes 31d disposed along the third hexagonal shape are illustrated to be disposed at an outermost side, but are not limited thereto, and the inlet holes 31 may also be disposed along two, four, or more hexagonal shapes having different areas. Although not illustrated in the drawings, the plurality of inlet holes 31 may also be disposed along concentric circles having different diameters around the center point CP of the central inlet hole 31a.

Referring to FIGS. 2 to 6, the inlet part 3 may include a plurality of partition walls 32.

The partition walls 32 partition the inlet holes 31. The partition walls 32 are connected to partition the inlet holes 31. The partition walls 32 may be individually formed and coupled to be connected to each other through a process such as a welding process. The partition walls 32 may also be integrally formed through a process such as an injection molding process.

The partition walls 32 may be disposed to surround the inlet holes 31. In this case, each of the inlet holes 31 may be disposed to be surrounded by N partition walls 32 (N is an integer greater than two). For example, each of the inlet holes 31 may be disposed to be surrounded by six partition walls 32. In this case, N is six, and the partition walls 32 surrounding each of the inlet holes 31 may have a hexagonal shaped cross section and may be formed in an entirely hexagonal pyramid shape. Although not illustrated in the drawings, the inlet holes 31 may be disposed to be surrounded by three, four, five, seven, or more partition walls 32. The inlet holes 31 may also be disposed to be surrounded by the partition walls 32 having circular shaped cross sections and formed in an entirely truncated cone shape. As described above, when compared to the modified embodiments, since the embodiment in which each of the inlet holes 31 is disposed to be surrounded by six partition walls 32 is realized in a honeycomb structure, the embodiment can be realized in a stronger structure. N partition walls 32 surrounding each of the inlet holes 31 may be connected at an included angle 32A of 120° (illustrated in FIG. 6). Accordingly, the partition walls 32 surrounding each of the inlet holes 31 may have a regular hexagonal shaped cross section. Accordingly, the partition walls 32 may be formed in a stronger structure. The included angle 32A is based on lower ends positioned at a lowermost side of the partition walls 32 in the vertical direction (the Z-axis direction).

The partition walls 32 may be obliquely formed such that distances between the facing partition walls 32 increase as the facing partition walls 32 extend downward. Accordingly, the partition walls 32 may be formed such that an area of the inlet 311 of each of the inlet holes 31 is greater than that of the outlet 312 thereof Each of the partition walls 32 may be obliquely formed outward. In this case, the partition walls 32 may be obliquely formed outward from the center point CP of the central inlet hole 31a.

The partition walls 32 disposed further outward may be obliquely formed outward at larger angles. Angles of the partition walls 32 are angles at which the partition walls 32 are inclined outward with respect to the vertical direction (the Z-axis direction). For example, the first partition wall 32a (illustrated in FIG. 4) surrounding the central inlet hole 31a may be obliquely formed outward at the first angle C1 (illustrated in FIG. 4), and the second partition wall 32b (illustrated in FIG. 4) disposed outward from the first partition walls 32a may be obliquely formed outward at the second angle C2 (illustrated in FIG. 4). The second angle C2 may be larger than the first angle C1. Accordingly, in the gas detection apparatus 1 according to the inventive concept, since the partition walls 32 disposed further outward may be obliquely formed further outward, an area of the inlet 311 each of the inlet holes 31 may be greater than that of the outlet 312 thereof. Accordingly, in the gas detection apparatus 1 according to the inventive concept, since a gas may be introduced from a wider region of the detection space 10, an amount of gas introduced from the inlets 311 can further increase.

Distances 32H (illustrated in FIG. 4) from lower ends 321 (illustrated in FIG. 4) of the partition walls 32 to the main body 2 may be the same in the vertical direction (the Z-axis direction). That is, as illustrated in FIG. 4, the distances 32H from the lower ends 321 to the upper ends 322 of the partition walls 32 may be the same. The lower end 321 is a portion positioned at a lowermost side of the partition wall 32, and the upper end 322 is a portion positioned at an uppermost side of the partition wall 32 in the vertical direction (the Z-axis direction). Accordingly, as illustrated in FIG. 3, a lower surface of the inlet part 3 facing the detection space 10 may be formed to be flat. In a case in which the distances 32H from the lower ends 321 to the upper ends 322 are the same and the partition walls 32 disposed further outward are obliquely formed further outward, lengths of the partition walls 32 disposed further outward may be longer.

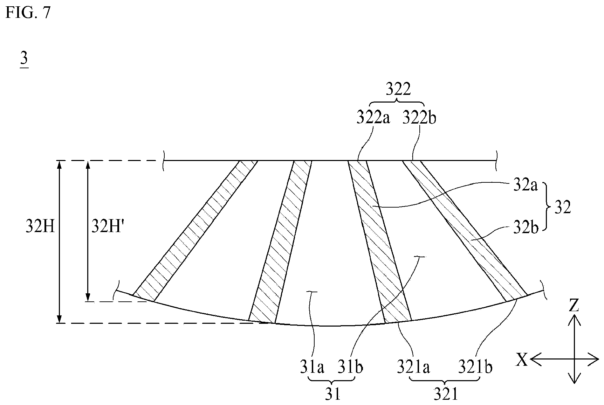

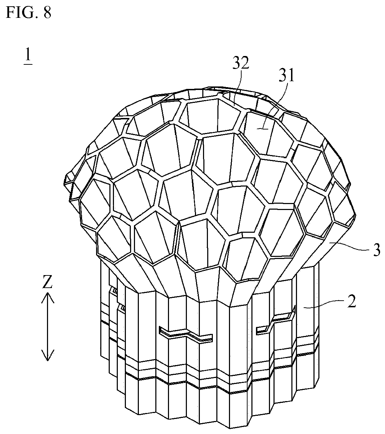

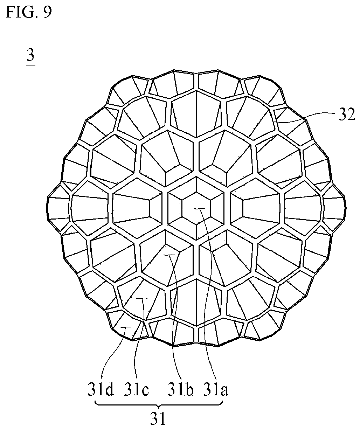

Referring to FIGS. 7 to 10, in a gas detection apparatus 1 according to the modified embodiment of the inventive concept, the distances 32H from the lower ends 321 of the partition walls 32 disposed further inward to the main body 2 may be longer in the vertical direction (the Z-axis direction). The term "outward" is an opposite direction of the term "inward." That is, as illustrated in FIG. 7, the distances 32H between the lower ends 321 and the upper ends 322 of the partition walls 32 disposed further inward may be longer. For example, the distances 32H between lower ends 321a and upper ends 322a of the first partition walls 32a may be greater than distances 32H' between lower ends 321b and upper ends 322b of the second partition walls 32b disposed further outward as compared to the first partition walls 32a.

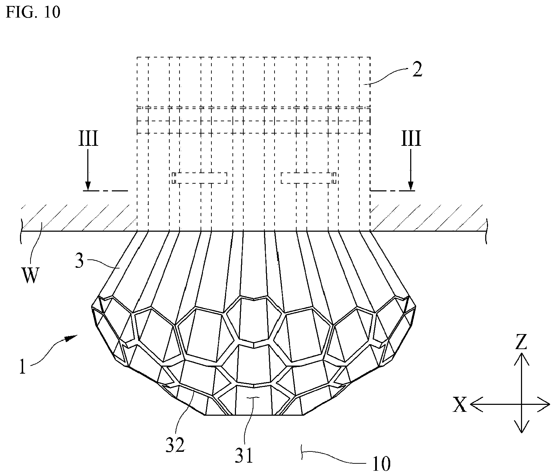

Accordingly, as illustrated in FIG. 10, a lower surface of the inlet part 3 facing the detection space 10 may be convexly formed downward. In this case, a portion corresponding to the central inlet hole 31a disposed at an innermost position in the inlet part 3 may protrude downward to have a longest length, and portions disposed outward from the central inlet hole 31a may protrude downward to have shorter lengths which gradually decrease. The lower surface of the inlet part 3 facing the detection space 10 may also be formed as a curved surface which is convex downward.

In the above-described gas detection apparatus 1 according to the modified embodiment of the inventive concept, since the lower surface of the inlet part 3 is formed to be convex downward, areas of inlets 311 of the inlet holes 31 may be wider. Accordingly, the gas detection apparatus 1 according to the modified embodiment of the inventive concept may increase an amount of gas introduced through the inlets 311. In addition, the gas detection apparatus 1 according to the modified embodiment of the inventive concept may be formed such that the inlets 311 disposed further outward may be formed further outward. Accordingly, the gas detection apparatus 1 according to the modified embodiment of the inventive concept may be formed so that a gas may be introduced from a wider region of the detection space 10 through the inlets 311.

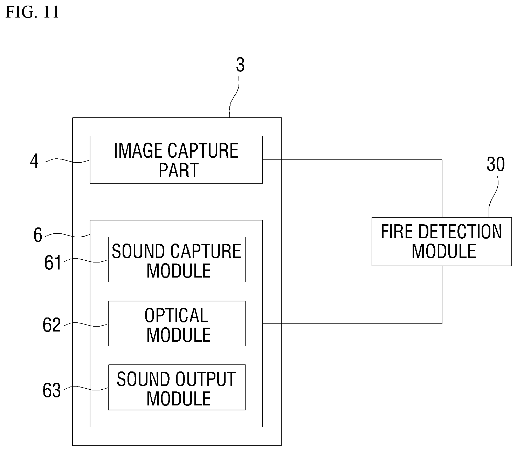

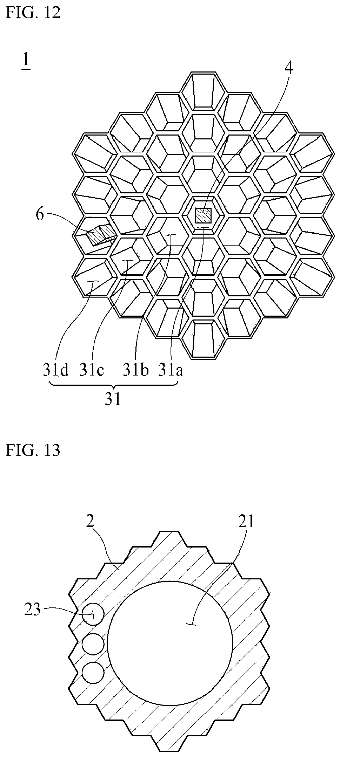

Referring to FIGS. 11 and 12, a gas detection apparatus 1 according to another inventive concept may include an image capture part 4.

The image capture part 4 is for capturing an image of the detection space 10. The image capture part 4 may capture an image of the detection space 10 to obtain image information of at least one of a static image and moving images. The image capture part 4 may provide the obtained image information to the fire detection module 30. The fire detection module 30 determines whether an emergency situation such as an outbreak of fire occurs. The fire detection module 30 may receive gas measurement data related to a result in which components of gases are analyzed from the gas sensor 20. The fire detection module 30 may use the gas measurement data to primarily determine whether an emergency situation such as an outbreak of fire occurs, and use the image information to secondarily determine whether the emergency situation occurs.

For example, the fire detection module 30 may use gas measurement data and image information to determine whether an emergency situation occurs through the following process.

First, when contents of components related to an outbreak of fire in gas measurement data received from the gas sensor 20 are greater than predetermined reference values, the fire detection module 30 may primarily determine that a fire has broken out in the detection space 10. The preset reference values may be content values of a component related to an outbreak of fire when the fire breaks out, and may be stored in the fire detection module 30 by a user.

Here, the preset reference values are reference values for determining whether a fire breaks out. For example, the preset reference values may include a value of an amount of gas detected by the gas sensor 20 in a stable state. However, the inventive concept is not limited thereto, and the preset reference values may be values which are arbitrarily set by the user.

According to one embodiment, in a case in which a change in environment of the detection space 10 occurs and gas component information of a stable state is changed, the fire detection module 30 may use the gas component information to correct the reference value.

Next, the fire detection module 30 may use image information to secondarily determine whether the fire actually has broken out in the detection space 10. In this case, the fire detection module 30 may determine whether the fire has broken out in the detection space 10 by comparing the image information provided by the image capture part 4 and a reference image. The reference image may be an image of a normal state of the detection space 10 and may be prestored in the fire detection module 30 by the user. The image information and the reference image may be thermal images in which temperature profiles are displayed in color.

Next, in a case in which it is determined that the fire does not break out from the image information, the fire detection module 30 may output a message, a symbol, or the like, which informs of an abnormality occurring in the gas sensor 20, to a display apparatus (not shown). In a case in which it is determined that the fire has broken out from the image information, the fire detection module 30 may output a message, a symbol, or the like, which informs of the outbreak of fire, to the display apparatus. In this case, the fire detection module 30 may also output a warning sound through a speaker, or the like.

As described above, the fire detection module 30 may use the gas measurement data to primarily determine whether the emergency situation occurs, and may use the image information to secondarily determine whether the emergency situation occurs. The gas sensor 20 may provide analysis information to the fire detection module 30 through at least one of wire communication and wireless communication. The image capture part 4 may also provide the gas measurement data to the fire detection module 30 through at least one of wire communication and wireless communication. The gas detection apparatus 1 according to another inventive concept may also include the fire detection module 30. The gas detection apparatus 1 according to another inventive concept may also include the gas sensor 20. Here, the plurality of gas sensors 20 may be provided in the gas detection apparatus 1, and the plurality of gas sensors 20 may detect different kinds of gas components.

The image capture part 4 may be positioned inside the inlet part 3. In this case, the image capture part 4 may be inserted into any one of the inlet holes 31 formed in the inlet part 3. The image capture part 4 may be inserted into the corresponding inlet hole 31 and coupled to the partition walls 32 surrounding the corresponding inlet hole 31.

The image capture part 4 may be inserted into the central inlet hole 31a among the inlet holes 31. Since the central inlet hole 31a among the inlet holes 31 is vertically disposed downward, the gas detection apparatus 1 according to another inventive concept may be formed such that the image capture part 4 may capture an image of a field of view (FOV) including a vertically lower side of the inlet part 3 so as to obtain image information. In addition, the gas detection apparatus 1 according to another inventive concept may reduce the extent to which the FOV of the image capture part 4 is covered by the partition walls 32.

Referring to FIGS. 11 to 15, the main body 2 may include wire holes 23 (illustrated in FIG. 13).

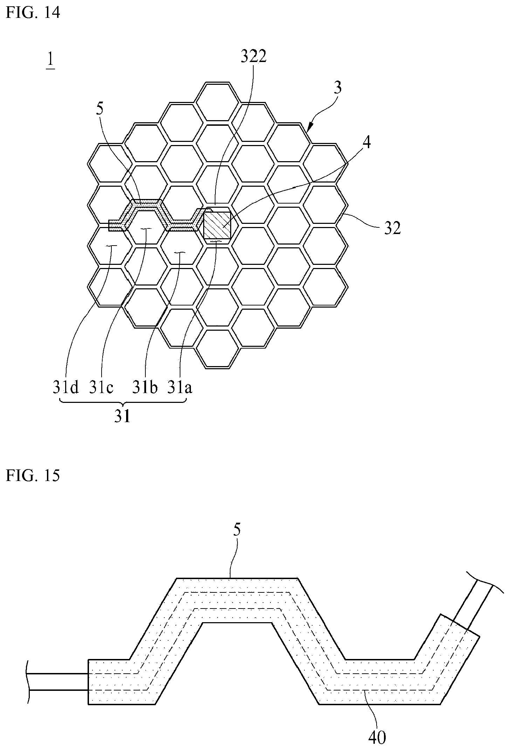

The wire hole 23 is for accommodating a wire 40 (illustrated in FIG. 15). The wire hole 23 may be disposed at a position spaced outward from the transfer hole 21 (illustrated in FIG. 13). Accordingly, since the gas detection apparatus 1 according to another inventive concept is formed to reduce an area in which the wire 40 covers the transfer hole 21 and the inlet holes 31, the extent to which an amount of introduced or transferred gas is reduced due to the wire 40 may be reduced.

The wire hole 23 may be formed to pass through the main body 2 in the vertical direction (the Z-axis direction). In FIG. 13, although two or three wire holes 23 are formed in the main body 2, the wire holes 23 are not limited thereto, and one, two, four or more wire holes 23 may also be formed in the main body 2. The wire hole 23 may be formed in an entirely cylindrical shape, but is not limited thereto, and may also be formed in another shape such as a polygonal prism shape as long as the wire hole 23 is capable of accommodating the wire 40. Since the wire 40 is withdrawn to an outside of the main body 2 through the wire hole 23, the wire 40 may be connected to a power source (not shown), a communication device (not shown), and the like in the ceil W (illustrated in FIG. 3).

Among the inlet holes 31, the wire hole 23 may be positioned at an upper side of the inlet holes 31 disposed at an outermost side. In this case, when the image capture part 4 is inserted into the central inlet hole 31a, the wire 40 connected to the image capture part 4 has to be installed to extend to the third inlet hole 31d (illustrated in FIG. 12) disposed at an outermost side from the central inlet hole 31a (illustrated in FIG. 12) and to be inserted into the wire hole 23. Accordingly, when the wire 40 linearly extends from the central inlet hole 31a to the third inlet hole 31d, since parts of inlet holes 31b and 31c positioned between the central inlet hole 31a and the third inlet hole 31d are covered by the wire 40, a gas flow may be reduced and damage such as corrosion may occur at the wire 40 in a case in which a gas which is corrosive, harmful, or the like flows through the inlet holes 31b and 31c.

For preventing damage, the gas detection apparatus 1 according to another inventive concept may include a protection part 5 (illustrated in FIG. 14).

The protection part 5 is for protecting the wire 40 connected to the image capture part 4. The protection part 5 may be coupled to upper ends of the partition walls 32. The protection part 5 may be formed in a shape corresponding to the upper ends of the partition walls 32. For example, as illustrated in FIG. 14, the protection part 5 may be formed in a shape which is bent so as to correspond to upper ends 322 of the partition walls 32 positioned between the partition wall 32 surrounding the central inlet hole 31a and the partition wall 32 surrounding the third inlet hole 31d. Accordingly, the wire 40 connected to the image capture part 4 may be bent along the upper ends 322 of the partition walls 32 by the protection part 5 and inserted into the wire hole 23.

Accordingly, in the gas detection apparatus 1 according to another inventive concept, even in a case in which the wire 40 connected to the image capture part 4 is installed to extend from the central inlet hole 31a to the third inlet hole 31d and to be inserted into the wire hole 23, the wire 40 may be formed to be bent along the upper ends 322 of the partition walls 32 using the protection part 5. Accordingly, in the gas detection apparatus 1 according to another inventive concept, since an area in which the wire 40 connected to the image capture part 4 convers the inlet holes 31b and 31c positioned between the central inlet hole 31a and the third inlet hole 31d is reduced, a gas may fluently flow through the inlet holes 31b and 31c.

In addition, in the gas detection apparatus 1 according to another inventive concept, since the wire 40 connected to the image capture part 4 is accommodated in the protection part 5, a gas flowing through the inlet holes 31b and 31c may be prevented from coming into direct contact with the wire 40 connected to the image capture part 4. Accordingly, in the gas detection apparatus 1 according to another inventive concept, damage such as corrosion of the wire 40 connected to the image capture part 4 due to a gas which is corrosive, harmful, or the like may be prevented.

Although the image capture part 4 has been described above on the basis of the embodiment in which the image capture part 4 is positioned in the central inlet hole 31a, the image capture part 4 may have the same operational effects even in a case in which the image capture part 4 is positioned in any one of the inlet holes 31b and 31c positioned under the transfer hole 21 other than the central inlet hole 31a. In this case, a length of the protection part 5 may be shorter when compared to the case in which the image capture part 4 is positioned in the central inlet hole 31a. Meanwhile, the protection part 5 may be formed of a material having a high corrosion resistance. The protection part 5 may also be formed of a material having a high thermal resistance. For example, the protection part 5 may be formed of aluminum, stainless steel, or the like.

Referring to FIGS. 11 to 16, the gas detection apparatus 1 according to another inventive concept may include a peripheral part 6 (illustrated in FIG. 11).

The peripheral part 6 is for identifying an emergency situation or informing of the emergency situation. The peripheral part 6 may include at least one among a sound capture module 61, an optical module 62, and a sound output module 63.

The sound capture module 61 is for capturing a sound. For example, the sound capture module 61 may be a microphone. The fire detection module 30 may use a sound provided by the sound capture module 61 to identify whether an emergency situation occurs. The sound capture module 61 may provide the captured sound to the fire detection module 30 through at least one of wire communication and wireless communication.

The optical module 62 is for emitting light. For example, the optical module 62 may be a light-emitting diode (LED). When it is determined that an emergency situation has occurred, the fire detection module 30 may control the optical module 62 such that the optical module 62 emits light. The fire detection module 30 may control the optical module 62 through at least one of wire communication and wireless communication.

The sound output module 63 is for outputting a sound. For example, the sound output module 63 may be a speaker. When it is determined that an emergency situation has occurred, the fire detection module 30 may control the sound output module 63 such that the sound output module 63 outputs a sound. The fire detection module 30 may control the sound output module 63 through at least one of wire communication and wireless communication.

The peripheral part 6 may also include only one among the sound capture module 61, the optical module 62, and the sound output module 63. The peripheral part 6 may also include two or more among the sound capture module 61, the optical module 62, and the sound output module 63. In a case in which the peripheral part 6 includes two or more among the sound capture module 61, the optical module 62, and the sound output module 63, the sound capture module 61, the optical module 62, and the sound output module 63 may be inserted into the different inlet holes 31.

The peripheral part 6 may be positioned in the inlet part 3. In this case, among the inlet holes 31 formed in the inlet part 3, the peripheral part 6 may be inserted into the inlet hole 31 disposed at an outermost side. Accordingly, the gas detection apparatus 1 according to another inventive concept is formed such that, among the inlet holes 31, an inlet hole 31 in which an amount of the introduced gas is relatively small is used as a space for accommodating the peripheral part 6. Accordingly, in the gas detection apparatus 1 according to another inventive concept, since a space accommodating the peripheral part 6 is removed in the detection space 10, space utilization may be improved and the extent to which the peripheral part 6 affects detection performance for detecting an emergency situation may be reduced.

The peripheral part 6 may be positioned in the inlet hole 31 positioned under the wire hole 23. Accordingly, since the wire connected to the peripheral part 6 is withdrawn to an outside of the main body 2 through the wire hole 23 even without the protection part 5, the wire may be connected to a power source (not shown), a communication device (not shown), and the like in the ceil W (illustrated in FIG. 3). In a case in which the inlet hole 31, in which the peripheral part 6 is positioned, and the wire hole 23 are spaced apart from each other, the gas detection apparatus 1 according to another inventive concept may further include the protection part 5 configured to accommodate the wire connected to the peripheral part 6.

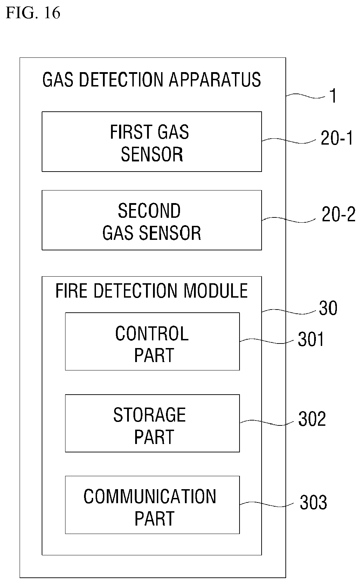

Referring to FIG. 16, a gas detection apparatus 1 according to still another inventive concept may include a first gas sensor 20-1, a second gas sensor 20-2, and a fire detection module 30, and determine whether a fire breaks out in the detection space 10 through the fire detection module 30.

The first gas sensor 20-1 and the second gas sensor 20-2 may be installed in the installation hole 22 (illustrated in FIG. 2) of the gas detection apparatus 1 according to the inventive concept, the first gas sensor 20-1 may detect a first gas generated in the detection space 10, and the second gas sensor 20-2 may detect a second gas which is generated in the detection space 10 and different from the first gas. Here, it is illustrated in FIG. 16 that the gas detection apparatus 1 according to still another inventive concept includes two gas sensors including the first gas sensor 20-1 and the second gas sensor 20-2, but the gas detection apparatus 1 is not limited thereto, and three or more gas sensors or only one gas sensor may be used according to a user's requirements or environments of use.

In addition, in the gas detection apparatus 1 according to still another inventive concept, it is illustrated that the first gas sensor 20-1 and the second gas sensor 20-2 detect the different first gas and second gas, but the first gas sensor 20-1 and the second gas sensor 20-2 are not limited thereto, and may be set to detect the same kind of gas according to a situation so as to improve gas detecting reliability.

The fire detection module 30 may receive gas measurement data of the first gas and the second gas detected by the first gas sensor 20-1 and the second gas sensor 20-2, analyze the received gas measurement data to determine whether a fire breaks out, and estimate an ignition material. To this end, the fire detection module 30 may include a control part 301, a storage part 302, and a communication part 303.

The fire detection module 30 may be wired or wirelessly connected to the first gas sensor 20-1 and the second gas sensor 20-2 through the communication part 303, and may receive the gas measurement data from the first gas sensor 20-1 and the second gas sensor 20-2.

Here, the gas measurement data is information data of the first gas and the second gas detected by the first gas sensor 20-1 and the second gas sensor 20-2. For example, the gas measurement data may include information of amounts of first gas and second gas and information of components of the first gas and the second gas, and the fire detection module 30 may receive the gas measurement data from the first gas sensor 20-1 and the second gas sensor 20-1 through wire communication or wireless communication such as Bluetooth or Wi-Fi. However, still another inventive concept is not limited thereto.

The fire detection module 30 may analyze the gas measurement data using the control part 301 to estimate a reference time, use analysis information for a preset time period from the reference time to determine whether a fire breaks out, and estimate an ignition object. Here, the reference time is an estimated time at which a fire has broken out.

In one embodiment, the control part 301 may estimate a time, at which amounts of the first gas and the second gas included in the gas measurement data received from the first gas sensor 20-1 and the second gas sensor 20-2 are greater than preset reference values, as the reference time.

In another embodiment, in a case in which the first gas sensor 20-1 and the second gas sensor 20-2 detects a gas which may be generated when a fire breaks out, the control part 301 may estimate a time at which the gas which may be detected when the fire breaks out is detected as a reference time. For example, in a case in which a gas which may be detected when a fire breaks out is carbon monoxide (CO), a time at which CO is detected by the first gas sensor 20-1 and the second gas sensor 20-2 may be estimated as a reference time.

In still another embodiment, the control part 301 may set a predetermined time, and may set each time at which the predetermined time passes from a time at which the gas detection apparatus 1 according to still another inventive concept starts to detect a gas as a reference time. For example, the control part 301 may set 30 minutes as the predetermined time, and in a case in which a time at which the gas detection apparatus 1 according to still another inventive concept starts to detect a gas is 9 A.M., the control part 301 may estimate each of 9:30 A.M., 10:00 A.M., and 10:30 A.M., at which 30 minutes have sequentially passed from 9:00 A.M., as the reference time. However, the reference time of still another inventive concept is not limited thereto, and any method in which the reference time may be set may be applied to set the reference time.

When the control part 301 analyzes gas measurement data to determine whether a fire breaks out, the control part 301 may determine that a fire has broken out only in a case in which amounts of the first gas and the second gas are greater than the preset reference values and gas components of the first gas and the second gas include gas components which are generated when a fire breaks out. For example, in a case in which amounts of the first gas and the second gas are greater than the preset reference values, but gas components are not gas components which may be generated when a fire breaks out, the control part 301 may determine that a fire has not broken out. Through this process, an error in which the gas detection apparatus 1 according to the inventive concept determines that a fire breaks out, due to an excessive amount of gas may be prevented.

When the control part 301 estimates an ignition material using gas measurement data measured for a preset time period from the reference time estimated through the above-described method, the control part 301 may estimate the ignition material using only the gas measurement data measured for the preset time period from the reference time among gas measurement data. For example, the control part 301 may analyze gas measurement data only measured for six seconds from the estimated reference time. However, the gas detection apparatus 1 according to still another inventive concept is not limited thereto, and the ignition material may be estimated by considering various factors including the gas measurement data.

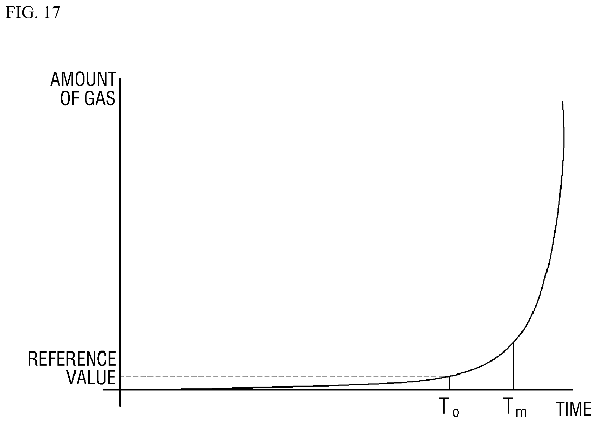

Referring to FIG. 17, in a case in which a fire has broken out, an ignition material generates smoke including a gas while being burnt, but since a fire spreads within the detection space 10 when a certain time passes, there is a difficulty in estimating the exact ignition material due to a large amount of gas. Accordingly, it is necessary to estimate the ignition material using a gas detected before a gas that is generated when a fire spreads from the ignition material by which the fire initially has broken out is detected so as to estimate the source of ignition material.

Here, in the gas detection apparatus 1 according to still another inventive concept, although an area in which a fire is detected may be variously set according to a user's requirements or environments of use, and the like, in a case in which the fire detection area is calculated by considering regulations related to a fire, an effective fire detection area of one gas detection apparatus 1 may be set to 50 m² or 7 mX7 m. Here, in a respect in which a speed of smoke including a gas flowing along a ceil of a passageway is in a range of 0.5 to 1 m/s and a speed of the smoke flowing along a stairway of a passageway is in a range of 2 to 3 m/s is considered, six seconds may pass while a gas generated due to a fire which has broken out within the effective fire detection area reaches the gas detection apparatus 1.

That is, since a gas generated due to a fire which breaks out at an initial ignition material and spread to anther material may be detected by the first gas sensor 20-1 and the second gas sensor 20-2 only when six seconds have passed from the reference time (a time at which the gas generated when the fire breaks out is detected by the first gas sensor 20-1 and the second gas sensor 20-2) estimated by the control part 301, the first gas sensor 20-1 and the second gas sensor 20-2 detects only the gas which is generated by the initial ignition material for about six seconds.

Accordingly, in a case in which the control part 301 estimates a time, at which a gas generated when a fire breaks out at an initial ignition material is detected by the first gas sensor 20-1 and the second gas sensor 20-2, as the reference time, sets the preset time to six seconds, and estimates the ignition material using gas components detected by the first gas sensor 20-1 and the second gas sensor 20-2 for six seconds from the reference time, since the ignition material may be estimated using only the gas generated by the initial ignition material, there is an advantage in that the ignition material can be more accurately estimated.

In one embodiment, the control part 301 may extract ignition material information matching gas components of the first gas and the second gas from ignition material information prestored in the storage part 302, and estimate an ignition material using the extracted ignition material information.

Here, the prestored ignition material information is information stored in the storage part 302 in which ignition materials, which are provided or providable in the detection space 10, are matched with information of gas components coming from the ignition material in a case in which a fire breaks out. However, in the gas detection apparatus 1 according to still another inventive concept, the fire detection module 30 may not include a separate storage part 302, and the ignition material information may be obtained from external server 8, which will be described below, and used thereafter.

For example, in a case in which a rate of gas components detected by the gas sensor 20 are carbon monoxide (CO), sulfur dioxide (SO₂) and carbon dioxide (CO₂) is 5:3:1, the control part 301 may extract information of an ignition material in which carbon monoxide (CO), sulfur dioxide (SO₂), and carbon dioxide (CO₂) are generated at a rate of 5:3:1 when a fire breaks out from gas component information stored in the storage part 302 and estimate the ignition material using the extracted ignition material information. Here, in a case in which the extracted ignition material information corresponds to a bed mattress, the control part 301 may estimate the bed mattress as the ignition material.

In another embodiment, the control part 301 may estimate an ignition material by performing a function of an embedded artificial intelligence (AI) related to ignition material information prestored in the storage part 302.

In still another embodiment, a gas detection apparatus 1 according to still another concept may include a temperature measurement part (not shown). The control part 301 may measure a heat release rate per unit time radiated from an ignition material through the temperature measurement part, and estimate the ignition material using the measured heat release rate.

In still another embodiment, the control part 301 may calculate a gas generation speed using amounts of gases detected by the first gas sensor 20-1 and the second gas sensor 20-2, and estimate an ignition material using the calculated gas generation speed. However, the gas detection apparatus 1 according to still another concept is not limited thereto, and any method of estimating an ignition material may be applied to the gas detection apparatus 1.

In one embodiment, in a case in which it is determined that a fire has broken out, the fire detection module 30 may output a warning signal of the outbreak of fire by using any one or more of the optical module 62 and the sound output module 63. For example, in a case in which a fire breaks out, the fire detection module 30 may output an audio guidance to inform of the outbreak of fire through a speaker or turn an LED on such that the user can recognize the outbreak of fire. However, the inventive concept is not limited thereto, the audio guidance output and the turning on of LED may be performed simultaneously.

In one embodiment, the fire detection module 30 may be inserted into any one inlet hole among the plurality of inlet holes formed in the gas detection apparatus 1 according to the inventive concept, and may be attached to any one portion on an outer surface of the gas detection apparatus 1. However, the gas detection apparatus 1 according to still another inventive concept is not limited thereto, may not include the fire detection module 30, may transmit gas measurement data to the external server 8, which will be described below, and perform a function, which is performed by the fire detection module 30, through the external server 8.

The storage part 302 may use gas measurement data received from the first gas sensor 20-1 and the second gas sensor 20-2 so as to store total ignition gas information including an ignition material, an ignition gas generation distribution, an ignition position, a detection time for which a gas flows from the ignition position to the gas detection apparatus 1, and a detected amount (ppb/ppm) per gas.

In one embodiment, the fire detection module 30 may calculate a speed of an outbreak of fire using an amount of gas measured for a preset time period from a reference time. For example, the fire detection module 30 may calculate a gas speed using Graham's gas diffusion theory, and calculate a speed of an outbreak of fire on the basis of the gas speed. However, still another inventive concept is not limited thereto.

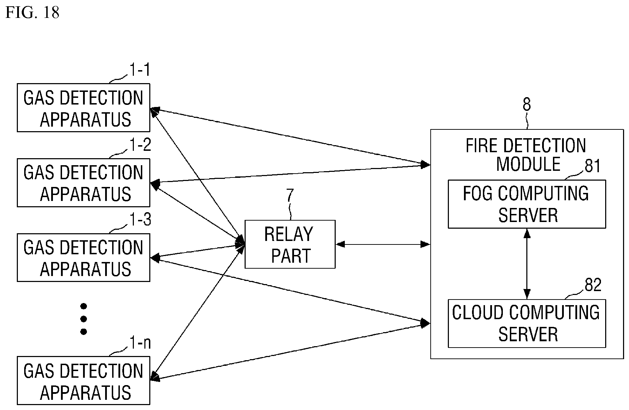

Referring to FIG. 18, in the gas detection apparatus 1 according to still another inventive concept, the gas detection apparatus 1 may be connected to a relay part 7 and the external server 8 through wire or wireless communication to build one network.

A plurality of gas detection apparatuses 1 according to still another inventive concept may be provided in the detection space 10 by considering the effective fire detection area, and plurality of gas detection apparatuses 1-1 to 1-n may be connected to the external server 8 by the relay part 7 through wire or wireless communication, or the plurality of gas detection apparatuses 1-1 to 1-n may be directly connected to the external server 8 through wire or wireless communication. For example, the plurality of gas detection apparatuses 1-1 to 1-n and the relay part 7 may be directly connected through a short-range wireless communication method, such as Bluetooth or Wi-Fi, or may be directly wired, and the relay part 7 may be connected to the external server 8 through a long-range wireless communication network. However, the gas detection apparatus 1 according to still another inventive concept is not limited thereto.

The relay part 7 may be wired or wirelessly connected to the plurality of gas detection apparatuses 1-1 to 1-n, build a roof computing server with the plurality of gas detection apparatuses 1-1 to 1-n, detect whether a fire breaks out within single buildings in which the plurality of gas detection apparatuses 1-1 to 1-n are installed, and warn the outbreak of fire when the fire breaks out.

The external server 8 may store, manage, and process information of the plurality of gas detection apparatuses 1-1 to 1-n installed in the detection space 10, gas measurement data received from the plurality of gas detection apparatuses 1-1 to 1-nm, information of whether a fire breaks out, and information of an ignition material.

The external server 8 may include a fog computing server 81 and a cloud computing server, and sequentially perform management and processing analysis information, information of whether a fire breaks out, and information of an ignition object received from the plurality of gas detection apparatuses 1 through the fog computing server 81 and the cloud computing server 82.

The fog computing server 81 may use gas measurement data and information of whether the fire breaks out and an ignition object received from the plurality of gas detection apparatuses 1-1 to 1-n so as to detect whether a fire breaks out and warn the outbreak of fire in a range of district or county, and the cloud computing server 82 may detect whether a fire breaks out and ward the outbreak of fire in a range of city or country.

That is, since the gas detection apparatus 1 according to still another inventive concept may determine whether a fire breaks out and estimate an ignition material by itself, and transmit information of a determination result of whether the fire breaks out and the estimated ignition material to the external server 8, there is an advantage in that a time for informing the outbreak of fire decreases when compared to a method in which the external server 8 determines whether a fire breaks out and estimates an ignition material.

In one embodiment, the external server 8 may use gas measurement data to re-determine whether a fire breaks out and re-estimate ignition material information regardless of information of whether a fire breaks out and ignition material received from the plurality of gas detection apparatuses 1-1 to 1-n. Accordingly, reliability of a determination result of whether a fire breaks out and an estimated ignition material can be improved, and it can be determined whether failures of the plurality of gas detection apparatuses 1-1 to 1-n occur.

In one embodiment, the external server 8 may estimate an ignition position in the detection space 10 using gas measurement data received from the plurality of gas detection apparatuses 1-1 to 1-n. For example, in a case in which a fire has broken out near the first gas detection apparatus 1-1 as illustrated in FIG. 18, although the plurality of gas detection apparatuses 1-1 to 1-4 positioned in the detection space 10 may determine whether the fire has broken out, an amount of gas detected by the first gas detection apparatus 1-1 may be greater than an amount of gas detected by the gas detection apparatuses 1-2 to 1-4. Accordingly, the external server 8 may estimate that an ignition position is near the first gas detection apparatus 1-1 according to information of the amount of gas, that is, a concentration of the gas.

In another embodiment, the external server 8 may estimate an ignition position using an order by which the plurality of gas detection apparatuses 1-1 to 1-4 transmits information of whether a fire breaks out and an ignition object. However, the gas detection apparatus 1 according to still another inventive concept is not limited thereto.

In one embodiment, the external server 8 may provide information of the gas sensor 20 needed to be additionally installed to the user using kinds of gas sensors 20 included in the plurality of gas detection apparatuses 1-1 to 1-n. For example, the external server 8 may collect information of an object provided within the detection space 10, and use the information of the connected object to determine a kind of gas which can be generated when a fire breaks out. Here, in a case in which the gas sensor 20 capable of detecting the determined kind of gas is not included in the plurality of gas detection apparatuses 1-1 to 1-n installed in the detection space 10, the external server 8 may provide information of the corresponding gas sensor 20 to the user.

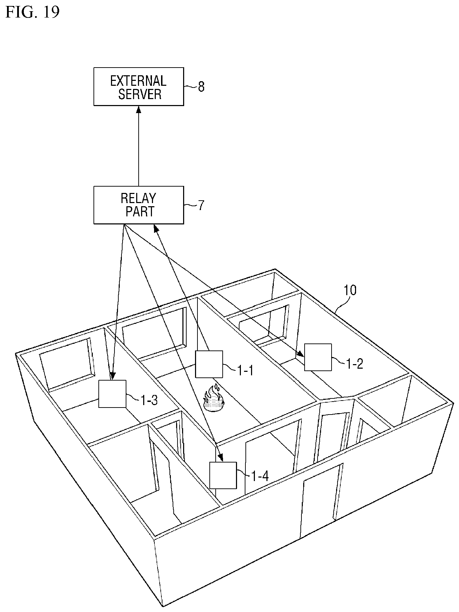

In one embodiment, the external server 8 may use gas measurement data received from each of the plurality of gas detection apparatuses 1-1 to 1-n to diagnose whether a failure occurs in the plurality of gas detection apparatuses 1-1 to 1-n, and provide a message for requiring replacement of the gas detection apparatus 1 which is estimated that a failure has occurred to the user. For example, in a case in which although a fire breaks out in the detection space 01, only one gas detection apparatus 1 among the plurality of gas detection apparatuses 1-1 to 1-n does not detect that the fire breaks out, the external server 8 may provide a message for requiring replacement of the gas detection apparatus 1 which cannot detect whether the fire breaks out. Hereinafter, a process in which information flows between the plurality of gas detection apparatuses 1-1 to 1-n, the relay part 7, and the external server 8 when a fire breaks out will be described with reference to FIG. 19.

Referring to FIG. 19, the plurality of gas detection apparatuses 1-1 to 1-4 may be installed in the detection space 10. For example, in a case in which a plurality of rooms are present as illustrated in FIG. 19, one gas detection apparatus 1 may be installed at each of the rooms by considering an effective fire detection area of the gas detection apparatus 1. Here, in a case in which a fire has broken out near the first gas detection apparatus 1-1, the first gas detection apparatus 1-1 may detect gases generated due to the outbreak of fire and generate gas measurement data, use the gas measurement data to determine whether the fire has broken out, and estimate an ignition material. When it is determined that the fire has broken out, the first gas detection apparatus 1-1 may output a warning signal of the outbreak of fire, and transmit information of whether the fire has broken out and an ignition material to the relay part 7.

The relay part 7 may transmit the information of whether the fire breaks out and the ignition material received from the first gas detection apparatus 1-1 to the external server 8 and the gas detection apparatuses 1-2 to 1-4 installed the other rooms. Here, the gas detection apparatuses 1-2 to 1-4 may output warning signals of the outbreak of fire, such as an audio guidance or turning on of LED, using the information of whether the fire breaks out and the ignition material received from the relay part 7.

In addition, the external server 8 may transmit information of whether the fire breaks out and the ignition material to a user terminal, and thus, the use can recognize a fact that the fire breaks out even in a case in which the user is not present near an ignition position.

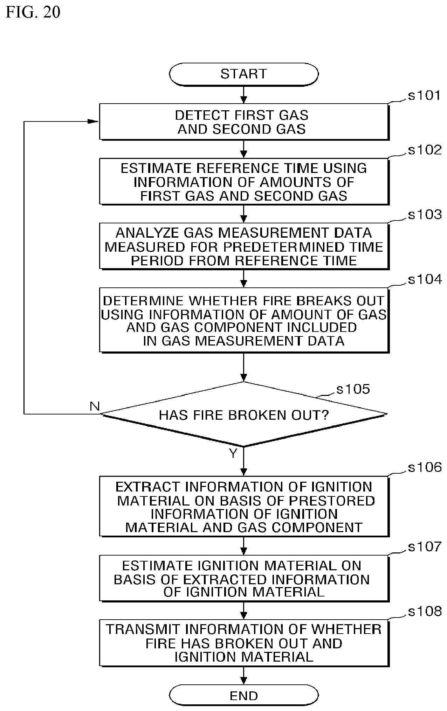

In one embodiment, when information is transmitted to or received from the plurality of gas detection apparatuses 1-1 to 1-n, the relay part 7, and the external server 8 through wire or wireless communication, the information may be transmitted or received on the basis of a blockchain. Hereinafter, a method of detecting ignition using the gas detection apparatus 1 according to still another inventive concept will be described with reference to FIG. 20.

Referring to FIG. 20, first, the first gas sensor 20-1 and the second gas sensor 20-2 detect a first gas and a second gas and generate gas measurement data (SI01), and the fire detection module 30 estimates a reference time using information of amounts of a first gas and a second gas included in the gas measurement data (S102).

The control part 301 analyzes the gas measurement data during a preset time period from the reference time estimated in operation S102 (S103), and thus determines whether a fire breaks out (S104).

Here, in a case in which it is determined that the fire has broken out (S105), the control part 301 extracts ignition material information using prestored ignition material information and the gas measurement data, and estimates an ignition material using the extracted ignition material information (S107).

Next, information of whether the fire has broken out which is determined in operation S104 and the ignition material information estimated in operation S107 is transmitted to the outside such as the relay part 7 and the external server 8 (S108).

When it is determined in operation S105 that the fire has not broken out, the operation returns to operation S101, and operations S101 to S105 are repeatedly performed to detect whether a fire breaks out in real time.

As a result, the following effect can be achieved.

Since the inventive concept is realized such that gases can be introduced from a wider region and an amount of the introduced gases can also be increased, it can be more accurately and quickly identified whether an emergency situation occurs.

The above-described inventive concepts are not limited to the above described embodiments and accompanying drawings, and it will be clear to those skilled in the inventive concepts that the inventive concepts may be variously substituted, changed, and modified without departing from the spirit of the inventive concepts.

## Claims

1. A gas detection apparatus comprising:
a main body in which a gas sensor is mounted;
an inlet part disposed under the main body to introduce a gas present in a detection space; and
a plurality of inlet holes formed to pass through the inlet part, wherein, in each of the inlet holes, an area of an inlet facing the detection space is greater than that of an outlet facing the main body.

2. The gas detection apparatus of claim 1, wherein:
the inlet part includes a plurality of partition walls configured to partition the inlet holes; and
the partition walls are formed obliquely such that distances between the facing partition walls increase as the facing partition walls extend downward, and
wherein the partition walls disposed further outward are obliquely formed outward at larger angles

3. The gas detection apparatus of claim 1, wherein:
the inlet part includes a plurality of partition walls configured to partition the inlet holes; and
distances from lower ends of the partition walls to the main body are the same in a vertical direction.

4. The gas detection apparatus of claim 1, wherein:
the inlet part includes a plurality of partition walls configured to partition the inlet holes; and
distances from lower ends of the partition walls disposed further inward to the main body are longer in a vertical direction.

5. The gas detection apparatus of claim 1, wherein:
the inlet part includes a plurality of partition walls configured to partition the inlet holes; and
the inlet holes are surrounded by N partition walls (N is an integer greater than two), and
wherein the N partition walls which surround the inlet holes are connected at an included angle of 120°.

6. The gas detection apparatus of claim 1, further comprising an image capture part configured to capture an image of the detection space,
wherein the image capture part is inserted into the inlet hole disposed at a center of the inlet holes to be positioned in the inlet part.

7. The gas detection apparatus of claim 1, further comprising a peripheral part including at least one among a sound capture module configured to capture a sound, an optical module configured to emit light, and a sound output module configured to output a sound,
wherein the peripheral part is inserted into the inlet hole disposed at an outermost side of the inlet holes to be positioned in the inlet part.

8. The gas detection apparatus of claim 1, wherein the main body includes:
a transfer hole formed to be connected to the inlet holes,
an installation hole formed to be connected to the transfer hole, and
a wire hole configured to accommodate a wire,
wherein the gas sensor is inserted into the installation hole such that a part of the gas sensor is positioned in the transfer hole, and
wherein the wire hole is positioned at a position spaced outward from the transfer hole.

9. The gas detection apparatus of claim 8, further comprising:
an image capture part configured to capture an image of the detection space; and
a protection part configured to protect the wire connected to the image capture part,
wherein the image capture part is positioned in any one of inlet holes positioned under the transfer hole, and
the protection part is coupled to upper ends of partition walls such that the wire connected to the image capture part is bent along the upper ends of the partition walls and inserted into the wire hole and is formed in a shape corresponding to the upper ends of the partition walls.

10. A gas detection apparatus comprising:
a first gas sensor configured to detect a first gas;
a second gas sensor configured to detect a second gas different from the first gas; and
a fire detection module configured to receive gas measurement data from the first gas sensor and the second gas sensor, analyze the gas measurement data to determine whether a fire breaks out, and estimate an ignition material,
wherein the fire detection module analyzes the gas measurement data to estimate a reference time, and estimates the ignition material using only the gas measurement data measured for a preset time period from the reference time among the gas measurement data as an analysis target.

11. The gas detection apparatus of claim 10, wherein the fire detection module:
estimates a time at which amounts of the first gas and the second gas are greater than preset reference values as the reference time; and
analyzes the gas measurement data measured for six seconds from the reference time to estimate the ignition material.

12. The gas detection apparatus of claim 10, wherein the fire detection module uses amounts of the first gas and the second gas and gas components to determines whether the fire breaks out, and determines that the fire has broken out in a case in which the amounts of gases are greater than preset reference values and the gas components include gas components generated when a fire breaks out.

13. The gas detection apparatus of claim 10, wherein the fire detection module:
extracts the ignition material information matching gas components of the first gas and the second gas of the prestored ignition material information; and
estimates the ignition material using the extracted ignition material information.

14. The gas detection apparatus of claim 10, wherein:
a plurality of gas detection apparatuses identical to the gas detection apparatus are provided in the detection space and are connected to an external server by a relay part through wire or wireless communication; and
the external server receives the gas measurement data from the plurality of gas detection apparatuses and estimates an ignition position using the gas measurement data.

15. An ignition detection method of determining whether a fire breaks out in a detection space and estimating an ignition material using a gas detection apparatus, the method comprising:
sensing, by a first gas sensor, a first gas;
sensing, by a second gas sensor, a second gas different from the first gas;
receiving, by a fire detection module, gas measurement data from the first gas sensor and the second gas sensor, and analyzing the gas measurement data to determine whether a fire breaks out; and
analyzing, by the fire detection module, the gas measurement data and estimating an ignition material,
wherein the estimating of the ignition material includes analyzing the gas measurement data to estimate a reference time, and estimating the ignition material using only the gas measurement data measured for a preset time period from the reference time among the gas measurement data as an analysis target.
